# EUROPEAN PATENT SPECIFICATION

(11) **EP 1 318 853 B1**
(45) Date of publication and mention of the grant of the patent: **01.06.2005**
(21) Application number: 01981158.7
(22) Date of filing: 20.09.2001
(51) Int. Cl.: A61N 1/05

(54) **PROBE FOR TREATMENT OF INCONTINENCE**
SONDE ZUR BEHANDLUNG VON INKONTINENZ
SONDE POUR LE TRAITEMENT DE L'INCONTINENCE

(30) Priority: 21.09.2000 NL 1016228
(43) Date of publication of application: 18.06.2003
(73) Proprietor: Fornix Medical Systems Holding B.V., 6641 SZ Beuningen (NL)
(72) Inventor: DIJKMAN, Gerrat, NL-7524 RJ Enschede (NL)
(74) Representative: Prins, Adrianus Willem, Mr. Ir.
(86) International application number: PCT/NL2001/000696
(87) International publication number: WO 2002/034328

(56) References cited:
- EP-A- 0 366 163
- FR-A- 2 767 481
- FR-A- 2 774 579
- GB-A- 2 301 287
- US-A- 3 640 284
- US-A- 5 483 832

## Description

The invention relates to a probe for treatment of urinary or faecal incontinence, which probe is substantially intended for intra-vaginal respectively intra-anal use and is provided on an outer surface with at least two electrodes, wherein the probe comprises a substantially hollow body of a flexible material, and is provided with means for measuring pressure in the body.

An intra-vaginal or intra-anal probe provided with electrodes is generally known in the treatment of incontinence, both for diagnostic and therapeutic purposes.

As diagnostic aid, the known probe is introduced intra-vaginally or intra-anally and the electric voltage is measured over the at least two electrodes. Such a measurement is known as electromyography (EMG). The result of a measurement with EMG gives an indication of the electrical activity of the sphincter vesicae (contractor of the bladder) respectively the sphincter ani (contractor of the anus) and of the pelvic floor as a whole. As therapeutic aid the known probe is applied to prickle and thus stimulate contraction of the muscle in question by applying a voltage over the at least two electrodes. In order to be able to assess whether a determined EMG activity also results in the desired contraction, and therefore whether eiectro-stimulation is a useful therapy, it must first be determined whether the relevant muscle is still able to contract. This final determination is carried out using an Intra-vaginal or intra-anal pressure probe, or by means of a vaginal or anal internal examination, the outcome of which is frequently unclear.

Working with different probes for diagnostic and therapeutic purposes has the effect of increasing costs, while the risk of infections is moreover increased.

It is noted that a probe of the type as described in the preamble is known from FR 2 774 579. The known probe is provided with a pair of ring shaped electrodes for electro stimulation and with a substantially hollow body for pressure measurement.

It is an object of the invention to provide a probe which is suitable for both therapeutic and diagnostic purposes in the field of electro-stimulation, and which is also suitable for performing pressure measurements.

This object is achieved, and other advantages gained, with a probe of the type specified in the preamble, wherein according to the invention, the electrodes extend over the flexible material are elongate and oriented in longitudinal direction of the body.

In a probe according to the invention the flexible walls of the hollow body act as transfer medium to transfer the pressure on the probe onto the interior of the probe, which pressure can be determined with the pressure measuring means.

By providing elongate electrodes which are oriented in longitudinal direction of the body the measuring surface is enlarged and the accuracy of positioning increased in advantageous manner. Application of elongate electrodes further enables the use of a larger number of electrodes than the two that are usual In practice, which provides additional options such as locally selective measurement and/or stimulation.

In a practical embodiment the probe comprises a front end cap and a rear end cap, the flexible material extending between the front end cap and the rear end cap forming the hollow body therein between.

Preferably the electrodes extend from the front end cap towards the rear end cap covering a substantial part of the flexible material in longitudinal direction of the body.

The probe is preferably provided with at least three electrodes, one of which serves as earth connection, so that the probe can be used to perform an EMG measurement without applying additional electrodes.

In a particularly advantageous embodiment the electrodes comprise electrically conducting silicone material. The body is for instance manufactured from silicone material or from polyurethane (PUR). Both materials are sufficiently flexible for the intended application and suitable for medical use.

The body of the probe according to the invention is advantageously obtained by extrusion.

In preference the body is generally cylinder-shaped, which, fits well in the bodily orifice(s) for which the probe is intended.

In an embodiment of a probe, the hollow body Is provided with a sealable opening, so that a bias can be adjusted in simple manner in the interior of the body.

The opening in the body can be sealed by a non-return valve.

The body of the probe according to the invention is advantageously obtained by extrusion.

The invention will be elucidated hereinbelow on the basis of an embodiment and with reference to the drawing, in which
Figure 1 shows a schematic view of a probe according to the invention; and
Figure 2 shows the probe according to figure 1 in longitudinal section.

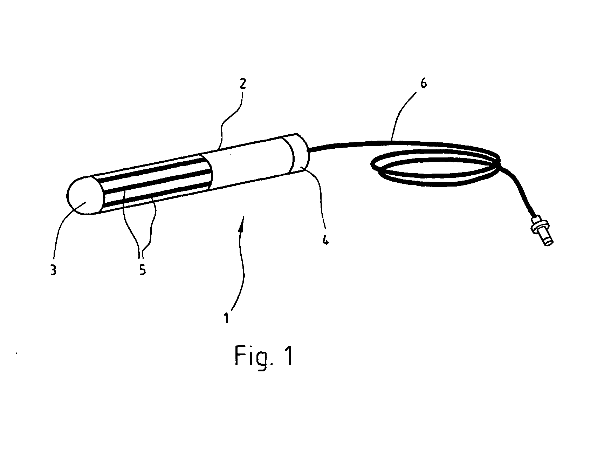

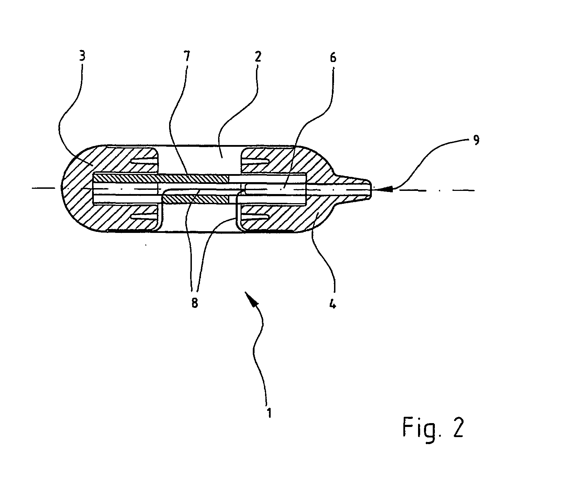

In the drawing figures 1 and 2 show a probe 1 intended for vaginal use. Probe 1 comprises a hollow body 2 which in this embodiment has a general cylinder shape and is manufactured from a flexible material suitable for medical application, for instance silicone material. An example of a suitable material is also designated in the field as silicone rubber. Cylinder 2 extends between a front end cap 3 and a rear end cap 4 of a suitable solid plastic material.

Electrodes 5 of an electrically conducting material suitable for medical application extend in longitudinal direction over the outer surface of cylinder 2. The above stated silicone material or silicone rubber is very suitable for this purpose. Electrodes 5 are connected to the cores 8 of a cable 6 guided through the rear end cap 4. It is noted that the probe according to the invention is suitable for connection to all electrical equipment known in the technical field for the purpose of diagnosis and treatment of incontinence, particularly by means of electro-stimulation.

Cylinder 2 is sealable by means of a non-return valve (not shown) to bring the interior of cylinder 2 to a desired bias. Arranged for this purpose in cable 6 is an air channel (not shown) which is connected to the interior of cylinder 2. The non-return valve serves to seal this air channel. Alternatively, the non-return valve can be arranged directly in opening 9 in rear end cap 4. No use is then made of cable 6.

A pressure sensor (not shown) is also provided for determining the pressure in the interior of cylinder 2. For this purpose the interior of cylinder 2 is placed in pressure connection, via the air channel in cable 6, with a separate apparatus (not shown) for measuring the pressure, wherein the pressure sensor is received in the apparatus. In the above stated alternative, In the absence of cable 6, the pressure sensor can be arranged in probe 1, for instance in rear end cap 4.

For strengthening purposes the end caps are mutually connected by a plastic rod 7, which also prevents undesired contact between electrodes 5. Rod 7 preferably takes a hollow form, so that electrical connecting wires 8 of electrodes 5 can be carried therein.

In practice electro-stimulation and measuring of EMG activity take place In the usual manner as generally applied in the field with known probes. The elongate electrodes 5 herein provide a number of significant advantages. Firstly, they provide a large measuring surface so that the relevant muscle can be measured/stimulated over a large part of the surface thereof. Owing to the large electrode surface, the probe can moreover be positioned correctly in the body of the patient by means of a fast and simple operation. In addition, the number of electrodes can be increased as desired, to for instance six, which provides the option of selective, local measurement/stimulation.

During a pressure measurement the body of the probe must be sealed. If desired, the probe can first be brought to an increased bias. The material of the hollow body 2 is preferably flexible for this purpose such that the diameter of hollow body 2 can be inflated to 1,5 times or even twice the diameter of the end caps. This has the advantage that the dimension of the probe can be adjusted to the dimension of the patient, which increases the effectiveness of the measurement/stimulation. The pressure which the patient exerts on the wall of hollow body 2 by contracting the relevant muscle results in a pressure increase in the interior of hollow body 2 which is measured by the pressure sensor.

The flexibility and resilience which are inherent to the probe according to the invention are maintained during use. This has the significant advantage in the treatment of incontinence that the muscle formation of the muscle under treatment in this case is additionally stimulated by the resistance encountered by this muscle during the exertion of pressure on the probe, which increases the effectiveness of the treatment.

It is finally noted that the probe according to the invention can be manufactured at a low cost price using the above stated materials. This makes the probe highly suitable for application as disposable article, i.e. the probe is then preferably intended for (re)use by one patient.

It is noted that in principle there is no difference between an intra-vaginal and an intra-anal probe, other than in shape and dimensions.

## Claims

1. Probe (1) for treatment of urinary or faecal incontinence, which probe is substantially intended for intra-vaginal respectively intra-anal use and is provided on an outer surface with at least two electrodes (5), wherein the probe (1) comprises a substantially hollow body (2) of a flexible material, and is provided with means for measuring pressure in the body (2), **characterized in that**, the said electrodes (5) extend over the flexible material, are elongate and oriented in longitudinal direction of the body (2)..

2. Probe as claimed in claim 1, wherein the electrodes are connected to the outside of the substantially hollow body (2) of a flexible material.

3. Probe as claimed in any one of claims 1-2, wherein the probe comprises a front end cap (3) and a rear end cap (4), the flexible material extending between the front end cap and the rear end cap forming the hollow body (2) therein between.

4. Probe as claimed in claim 3, wherein the electrodes extend from the front end cap towards the rear end cap covering a substantial part of the flexible material in longitudinal direction of the body (2).

5. Probe as claimed in any of the claims 1-4, wherein the probe (1) is provided with at least three electrodes.

6. Probe as claimed in any of the claims 1-5, wherein the body (2) is manufactured from silicone material and wherein the electrodes (5) comprise electrically conducting silicone material.

7. Probe as claimed in any of the claims 1-6, wherein the body (2) is obtained by extrusion.

8. Probe as claimed in any of the claims 1-7, wherein the body (2) has a generally cylindrical shape.

9. Probe as claimed in any of the claims 1-8, wherein the body (2) is provided with a sealable opening.

10. Probe as claimed in claim 9, wherein the opening can be sealed by a non-return valve.

## Patentansprüche

1. Sonde (1) zur Behandlung von Harn- oder Fäkal-Inkontinenz, wobei die Sonde im Wesentlichen für intra-vaginale bzw. intra-anale Verwendung dient und an einer äußeren Oberfläche mit wenigstens zwei Elektroden (5) versehen ist, wobei die Sonde (1) einen im Wesentlichen hohlen Körper (2) aus biegsamem Material aufweist, und mit Mitteln zum Messen des Drucks in dem Körper (2) versehen ist, **dadurch gekennzeichnet, dass** die Elektroden (5), die sich über das biegsame Material erstrecken, länglich sind und in der Längsrichtung des Körpers (2) ausgerichtet sind.

2. Sonde nach Anspruch 1, wobei die Elektrode mit der Außenseite des im Wesentlichen hohlen Körpers (2) aus biegsamem Material verbunden sind.

3. Sonde nach Anspruch 1 oder 2, wobei die Sonde eine vordere Endkappe (3) und eine hintere Endkappe (4) aufweist, wobei das biegsame Material sich zwischen der vorderen Endkappe und der hinteren Endkappe erstreckt unter Bildung des hohlen Körpers (2) dazwischen.

4. Sonde nach Anspruch 3, wobei die Elektroden, die sich von der vorderen Endkappe zu der hinteren Endkappe erstrecken, einen wesentlichen Abschnitt des biegsamen Materials in Längsrichtung des Körpers (2) bedecken.

5. Sonde nach einem der Ansprüche 1 bis 4, wobei die Sonde (1) mit wenigstens drei Elektroden versehen ist.

6. Sonde nach einem der Ansprüche 1 bis 5, wobei der Körper (2) aus einem Silikonmaterial gefertigt ist, und wobei die Elektroden (5) elektrisch leitendes Silikonmaterial aufweisen.

7. Sonde nach einem der Ansprüche 1 bis 6, wobei der Körper (2) durch Extrusion erhalten wird.

8. Sonde nach einem der Ansprüche 1 bis 7, wobei der Körper (2) eine im Wesentlichen zylindrische Form aufweisen.

9. Sonde nach einem der Ansprüche 1 bis 8, wobei der Körper (2) mit einer abdichtbaren Öffnung versehen ist.

10. Sonde nach Anspruch 9, wobei die Öffnung durch ein Rückschlagventil verschlossen werden kann.

## Revendications

1. Sonde (1) pour le traitement de l'incontinence urinaire ou fécale, laquelle sonde est sensiblement destinée à une utilisation intra-vaginale, respectivement intra-anale, et est équipée sur sa surface externe, d'au moins deux électrodes (5), dans laquelle la sonde (1) comprend un corps sensiblement creux (2) en une matière souple, et est équipée de moyens pour mesurer la pression dans le corps (2),
**caractérisée en ce que** lesdites électrodes (5) s'étendent sur la matière souple, sont allongées et orientées dans la direction longitudinale du corps (2).

2. Sonde telle que revendiquée dans la revendication 1, dans laquelle les électrodes sont reliées à l'extérieur du corps sensiblement creux (2) en matière souple.

3. Sonde telle que revendiquée dans l'une quelconque des revendications 1 - 2, dans laquelle la sonde comprend un capuchon d'extrémité avant (3) et un capuchon d'extrémité arrière (4), la matière souple s'étendant entre le capuchon d'extrémité avant et le capuchon d'extrémité arrière formant le corps creux (2) entre eux.

4. Sonde telle que revendiquée dans la revendication 3, dans laquelle les électrodes s'étendent du capuchon d'extrémité avant vers le capuchon d'extrémité arrière recouvrant une partie substantielle de la matière souple dans la direction longitudinale du corps (2).

5. Sonde telle que revendiquée dans l'une quelconque des revendications 1 - 4, dans laquelle la sonde (1) est équipée d'au moins trois électrodes.

6. Sonde telle que revendiquée dans l'une quelconque des revendications 1 - 5, dans laquelle le corps (2) est fabriqué à partir de matière en silicone et dans laquelle les électrodes (5) comprennent une matière en silicone électriquement conductrice.

7. Sonde telle que revendiquée dans l'une quelconque des revendications 1 - 6, dans laquelle le corps (2) est obtenu par extrusion.

8. Sonde telle que revendiquée dans l'une quelconque des revendications 1 - 7, dans laquelle le corps (2) présente une forme généralement cylindrique.

9. Sonde telle que revendiquée dans l'une quelconque des revendications 1 - 8, dans laquelle le corps (2) est muni d'une ouverture pouvant être fermée de façon étanche.

10. Sonde telle que revendiquée dans la revendication 9, dans laquelle l'ouverture peut être fermée de façon étanche par un clapet anti-retour.
